# FASCICULE DE BREVET EUROPEEN

(11) **EP 0 702 949 B1**
(45) Date de publication et mention de la délivrance du brevet: **17.12.1997**
(21) Numéro de dépôt: 95401384.3
(22) Date de dépôt: 14.06.1995
(51) Int. Cl.: A61K 7/13

(54) **Utilisation de gomme de guar dans des compositions non rincées pour la teinture des fibres kératiniques et procédé de teinture**
Verwendung des Guargummis in Präparaten zum Färben der Keratinfasern ohne Spühlung und Verfahren zur Färbung
Use of guar gum in non-rinsed compositions for dyeing keratinous fibers and dyeing process

(30) Priorité: 22.07.1994 FR 9409120
(43) Date de publication de la demande: 27.03.1996
(73) Titulaire: L'OREAL, 75008 Paris (FR)
(72) Inventeur: Samain, Henri, F-95170 Bievres (FR); Audousset, Marie-Pascale, F-92600 Asnieres (FR)
(74) Mandataire: Andral, Christophe André Louis

(56) Documents cités:
- EP-A- 0 531 738
- GB-A- 2 163 460
- GB-A- 2 254 341

## Description

La présente invention a pour objet l'utilisation de gomme de guar dans des compositions non rincées destinées à la teinture des fibres kératiniques et en particulier des fibres kératiniques humaines telles que les cheveux.

Il existe principalement deux types de coloration des fibres kératiniques. La coloration directe mettant en oeuvre des colorants directs et / ou des pigments qui sont des molécules colorées conférant aux fibres une couleur temporaire s'estompant après quelques shampooings et la coloration dite "coloration d'oxydation" mettant en oeuvre des précurseurs de colorants d'oxydation et un agent oxydant qui confère aux fibres une couleur tenace.

Ces deux types de coloration nécessitent l'application sur les fibres kératiniques de compositions contenant, dans un support approprié pour la teinture, les divers colorants directs et / ou pigments et / ou précurseurs de colorants d'oxydation en présence d'un agent oxydant. Ces compositions se présentent généralement sous la forme d'un liquide épaissi de façon à en faciliter l'application sur les fibres. Les divers agents épaississants utilisés dans de telles compositions sont en général choisis parmi les polymères acryliques éventuellement réticulés, les dérivés de cellulose, les gommes arabique, de guar, de Xanthane, de Carraghénanes, etc.

Après un temps de pose plus ou moins long permettant l'imprégnation des fibres par les colorants, ces compositions sont rincées pour éliminer le support de teinture. En effet, lorsque que ce type de composition n'est pas rincé, les cheveux ont du mal à sécher et lorsqu'ils sont secs, ils sont peu brillants, rêches, collants et de plus tachent fortement les tissus avec lesquels ils sont en contact (oreillers, serviettes). Cette technique présente donc l'inconvénient de demander du temps en imposant deux opérations distantes dans le temps qui sont la pose et le rinçage.

Dans la demande de brevet GB-A-0 163 460, on a décrit une composition de teinture à base du colorant direct 2-nitroparaphénylènediamine dont on a amélioré la solubilité dans l'eau par cosolubilisation avec un dérivé de Xanthine; cette composition de teinture peut être épaissie de façon connue, et parmi les épaississants conventionnels, on cite notamment une gomme de guar.

Dans la demande de brevet GB-A-1 254 341, on a proposé, pour obtenir des colorations capillaires à base de colorants acides qui soient résistantes aux lavages répétés, d'associer dans une composition de teinture que l'on rince après qu'on l'ait appliquée sur les cheveux, un colorant de type acide, un polymère cationique dont le taux d'azote est compris entre 0,5 et 3% et qui comprend une gomme de guar cationique, avec un agent tensio-actif spécifique choisi parmi les bétaines et les glutamates. Ladite composition permet d'éviter la formation d'un complexe entre le colorant de type acide et le polymère cationique, en l'occurrence la gomme de guar cationique, complexe qui empêche la pénétration du colorant acide dans le cheveu.

Dans la demande de brevet EP-A-0 531 738, on a décrit une composition de traitement des fibres kératiniques dont les effets conditionnants sont de longue durée après qu'elle ait été rincée, et qui est constituée de deux parties; la première renferme un solvant organique et un acide et peut renfermer un colorant direct, la seconde, un polymère cationique pouvant comprendre une gomme de guar cationique.

D'autre part, dans la demande de brevet européen EP-A-0072 298, sont décrites des compositions tinctoriales renfermant des colorants directs naturels, épaissies par un dérivé cationisé de gomme de guar. L'application de ces compositions sur les cheveux est suivie d'un rinçage, ce qui a pour conséquence d'éliminer la majorité des colorants, ce qui ne peut qu'accentuer la fugacité des colorations obtenues.

C'est afin de remédier à ces divers inconvénients que la demanderesse a découvert ce qui fait l'objet de l'invention.

L'invention a donc pour objet l'utilisation d'au moins une gomme de guar non-ionique et / ou d'au moins une gomme de guar modifiée par des groupements cationiques, comme seul agent épaississant, dans des compositions cosmétiques renfermant au moins un colorant direct et / ou au moins un pigment et / ou au moins un précurseur de colorant d'oxydation pour la coloration non rincée, directe ou d'oxydation, des fibres kératiniques et en particulier des fibres kératiniques humaines telles que les cheveux.

L'utilisation des gommes de guar selon l'invention permet de ne pas rincer les compositions tinctoriales. Les cheveux sèchent facilement et une fois secs, ils sont brillants, doux au toucher et ne tachent pratiquement pas les matériaux sur lesquels ils sont frottés. Les colorations obtenues sont en outre plus puissantes que lorsqu'une étape de rinçage suit l'application de la composition tinctoriale.

Dans ces conditions, il devient possible de teindre les fibres kératiniques et en particulier les fibres kératiniques humaines telles que les cheveux en n'employant qu'une seule étape qui est l'application de la composition tinctoriale. Ainsi, le temps passé par l'opérateur (coiffeur ou utilisateur) est très fortement réduit.

Les gommes de guar non-ioniques utilisables selon l'invention sont de préférence modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants tel que par exemple des oxydes de propylène avec la gomme de guar de façon à obtenir une gomme de guar modifiée par des groupements hydroxypropyle.

Le taux d'hydroxyalkylation varie de préférence de 0,4 à 1,2 et correspond au nombre de molécules d'oxyde d'alkylène consommé par le nombre de fonctions hydroxyle libres présentes sur la gomme de guar.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120 par la société MAYHALL.

Les gommes de guar modifiées par des groupements cationiques plus particulièrement utilisables selon l'invention sont des gommes de guar comportant des groupements cationiques trialkylammonium. De préférence, 2 à 30 % en nombre des fonctions hydroxyle de ces gommes de guar porte des groupements cationiques trialkylammonium. Encore plus préférentiellement, 5 à 20 % du nombre des fonctions hydroxyle de ces gommes de guar est branché par des groupements cationiques trialkylammonium.

Parmi ces groupements trialkylammonium, on peut tout particulièrement citer les groupements triméthylammonium et triéthylammonium.

Encore plus préférentiellement, ces groupements représentent de 5 à 20 % en poids par rapport au poids total de la gomme de guar modifiée.

Selon l'invention, on utilise de préférence une gomme de guar modifiée par du chlorure de 2,3-époxypropyl triméthylammonium.

Ces gommes de guar modifiées par des groupements cationiques sont des produits déjà connus en eux-mêmes et sont par exemple décrits dans les brevets US 3 589 578 et US 4 031 307. De tels produits sont par ailleurs vendus notamment sous les dénominations commerciales de JAGUAR C13 S, JAGUAR C 15, JAGUAR C 17 par la société MAYHALL.

Selon l'invention, les gommes de guar non-ioniques et / ou les gommes de guar modifiées par des groupements cationiques sont utilisées, en général, à raison de 0,1 à 3 % en poids par rapport au poids total de la composition tinctoriale et encore plus préférentiellement à raison de 0,25 à 2,5 % en poids.

Les colorants directs utilisables selon l'invention sont choisis parmi les colorants directs classiquement employés pour la coloration directe des fibres kératiniques. Parmi ceux-ci, on peut citer à titre d'exemple, les dérivés nitrés de la série benzénique, les colorants azoïques, les colorants anthraquinoniques, les indamines, indoanilines et indophénols, les colorants acides tels que ceux référencés au Color Index 3ème édition, les colorants naturels tels que la Lawsone. Ces colorants directs peuvent éventuellement porter des groupements sulfoniques ou cationiques de façon à améliorer leur solubilité dans le milieu utilisé pour la teinture.

Les pigments utilisables selon l'invention peuvent être choisis parmi les pigments minéraux ou organiques que l'on utilise de façon classique en cosmétique.

Parmi les pigments minéraux, on peut citer à titre d'exemple le dioxyde de titane (rutile ou anatase) éventuellement traité en surface et codifié dans le Color Index sous la référence CI 77891 ; les oxydes de fer noir, jaune, rouge et brun, codifiés sous les références CI 77499, 77492, 77491 ; le violet de manganèse (CI 77742) ; le bleu outremer (CI 77007) ; l'oxyde de chrome hydraté (CI 77289) ; le bleu ferrique (CI 77510).

Parmi les pigments organiques, on peut citer à titre d'exemple, le pigment yellow 3 vendu notamment sous la dénomination commerciale JAUNE COVANOR W 1603 par le société WACKHERR (CI 11710), le D & C red n° 19 (CI 45170), le D & C red n° 9 (CI 15585), le D & C red n° 21 (CI 45380), le D & C orange n° 4 (CI 15510), le D & C orange n° 5 (CI 45370), le D & C red n° 27 (CI 45410), le D & C red n° 13 (CI 15630), le D & C red n° 7 (CI 15850-1), le D & C red n° 6 (CI 15850-2), le D & C yellow n° 5 (CI 19140), le D & C red n° 36 (CI 12085), le D & C orange n° 10 (CI 45425), le D & C yellow n° 6 (CI 15985), le D & C red n° 30 (CI 73360), le D & C red n° 3 (CI 45430), le noir de carbone (CI 77266), et les laques à base de carmin de cochenille (CI 75470).

On peut également utiliser des pigments nacrés qui peuvent être notamment choisis parmi les pigments nacrés blancs tels que le mica recouvert d'oxyde de titane, l'oxyde de bismuth ; les pigments nacrés colorés tels que le mica titane avec des oxydes de fer, le mica titane avec du bleu ferrique ou de l'oxyde de chrome, le mica titane avec un pigment organique de type précipité, ainsi que ceux à base d'oxychlorure de bismuth.

Les précurseurs de colorants d'oxydation utilisables selon l'invention dans les compositions destinées à la coloration d'oxydation sont connus en eux-mêmes. On peut se référer plus particulièrement à ZVIAK, Sciences des traitements capillaires 1988, pages 235 à 287. Il s'agit plus particulièrement de diamines ou aminophénols comportant des groupements fonctionnels amino et hydroxy en position para ou ortho. Ces précurseurs de colorants d'oxydation, encore appelés bases d'oxydation, sont des composés incolores ou faiblement colorés qui une fois mélangés à des produits oxydants, au moment de l'emploi, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, choisis notamment parmi les métadiamines aromatiques, les métaaminophénols et les métadiphénols.

Les compositions tinctoriales peuvent également contenir des précurseurs indoliques générant des pigments de type mélanique sous l'action d'un agent oxydant. Ces précurseurs indoliques sont plus particulièrement décrits dans les demandes de brevet et brevets français FR-A-2 593 061, 2 593 062, 2 595 245, 2 606 636, 2 636 237 et les demandes de brevet européen EP-A-425 345, EP-A-424 261. Les précurseurs indoliques préférés sont choisis parmi le 5,6-dihydroxyindole et ses dérivés et les 6- et 7-monohydroxyindoles.

Le milieu approprié pour la teinture est généralement un milieu aqueux constitué par de l'eau ou un mélange d'eau et d'un solvant organique utilisé pour solubiliser les composés qui ne seraient pas suffisamment solubles dans l'eau.

Parmi ces solvants, on peut citer à titre d'exemple, les alcanols inférieurs en C₁-C₄, tels que l'éthanol et l'isopropanol ; le glycérol ; les glycols et éthers de glycols comme le 2-butoxyéthanol, le propylèneglycol, le monoéthyléther et le monométhyléther du diéthylèneglycol ; ainsi que les alcools aromatiques comme l'alcool benzylique ou le phénoxyéthanol ; les produits analogues et leurs mélanges. Selon l'invention, on utilise de préférence des alcools inférieurs en C₁-C₄ et tout particulièrement l'éthanol.

Lorsqu'ils sont présents, les solvants représentent de préférence 1 à 50 % en poids du poids total de la composition tinctoriale et encore plus préférentiellement de 5 à 40 % en poids.

Le pH de la composition appliquée sur les cheveux est de préférence compris entre 3 et 11. Il est ajusté à la valeur désirée au moyen d'agents alcalinisants habituellement utilisés en teinture des fibres kératiniques, tels que l'ammoniaque, les carbonates alcalins, les alcanolamines telles que par exemple les mono-, di- et triéthanolamines ainsi que leurs dérivés, les hydroxydes de sodium ou de potassium ou au moyen d'agents acidifiants classiques tels que des acides minéraux ou organiques comme par exemple les acides chlorhydrique, tartrique, citrique, lactique et orthophosphorique.

La composition tinctoriale peut en outre renfermer au moins un adjuvant utilisé habituellement en teinture des fibres kératiniques à condition que celui-ci ne modifie pas la consistance de la composition tinctoriale et n'entraîne pas un toucher désagréable des cheveux.

Parmi ces adjuvants, on peut citer les agents conservateurs, les agents séquestrants, les agents anti-oxydants, les parfums, les tensioactifs non-ioniques, les filtres solaires, etc.

La composition tinctoriale épaissie par la ou les gommes de guar utilisées selon l'invention se présente sous forme de gel plus ou moins consistant.

L'invention a également pour objet un procédé de teinture des fibres kératiniques et en particulier des fibres kératiniques humaines telles que les cheveux caractérisé par le fait qu'on applique sur ces fibres, une composition tinctoriale renfermant, au moins un colorant direct et / ou au moins un pigment et / ou au moins un précurseur de colorant d'oxydation, et au moins une gomme de guar non-ionique et / ou au moins une gomme de guar modifiée par des groupements cationiques telle que définie précédemment, puis on laisse sécher les fibres ou on les sèche à l'aide d'un sèche-cheveux sans les rincer. Lorsque les fibres sont sèches on peut les peigner ou y passer la main pour les délier.

Lorsque les compositions tinctoriales sont utilisées pour la coloration d'oxydation celles-ci sont mélangées au moment de l'emploi avec une composition contenant, dans un milieu approprié pour la teinture, au moins un agent oxydant.

L'agent oxydant est choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

La composition contenant l'agent oxydant peut également être épaissie et auquel cas elle ne contient pas d'autre agent épaississant que les gommes de guar telles que définies précédemment.

Le pH de la composition oxydante est tel qu'après mélange avec la composition tinctoriale, le pH de la composition résultante appliquée sur les fibres kératiniques est de préférence compris entre 3 et 11. Il est ajusté à la valeur désirée à l'aide d'agents acidifiants ou éventuellement alcalinisants bien connus de l'état de la technique, tels que décrits ci-dessus.

Le mélange de la composition tinctoriale et de la composition oxydante est ensuite appliqué sur les fibres kératiniques dans les mêmes conditions que précédemment.

Les exemples qui suivent sont destinés à illustrer l'invention.

### EXEMPLE 1 D'UTILISATION EN COLORATION DIRECTE

On prépare les compositions tinctoriales 1 et 2 suivantes :

### Composition 1 ne faisant pas partie de l'invention :

| | |
|---|---|
| - Lawsone | 0,5 g |
| - Polymère d'acide acrylique réticulé vendu sous la dénomination commerciale CARBOPOL 934 par la société GOODRICH | 1 g |
| - Ethanol | 50 g |
| - Eau déminéralisée q.s.p. | 100 g |

### Composition 2 faisant partie de l'invention

| | |
|---|---|
| - Lawsone | 0,5 g |
| **- Gomme de guar modifiée par des groupements cationiques vendue sous la dénomination commerciale JAGUAR C 13 S par la société MAYHALL** | **1 g** |
| - Ethanol | 50 g |
| - Eau déminéralisée q.s.p. | 100 g |

On applique ces compositions sur des mèches de cheveux européens préalablement lavés au shampooing, à raison d'environ 1g de composition par gramme de cheveux. On laisse les cheveux sécher naturellement puis on les délie avec un peigne.

Les cheveux teints avec la composition 1 utilisant un agent épaississant ne faisant pas partie de l'invention ont du mal à sécher et si l'on attend qu'ils soient secs, ils présentent un toucher désagréable et inacceptable. De plus les cheveux tâchent le papier sur lequel ils sont frottés.

A l'opposé, les cheveux teints avec la composition 2 utilisant une gomme de guar modifiée par des groupements cationiques selon l'invention sèchent facilement, ils présentent une coloration intense et ont un toucher agréable et naturel. Le passage de la mèche sur une feuille de papier ne laisse pratiquement pas de trace.

### EXEMPLE 2

On prépare la composition tinctoriale suivante :

| | |
|---|---|
| - Acid black 1 (référencé CI 20470 au Color Index 3ème édition) vendu par la société ZENECA COLOURS | 0,072 g |
| - Acid violet 43 (référencé CI 60730 au Color Index 3ème édition) vendu par la société TRICON COLORS | 0,072 g |
| - Orange Sulfacide JR Extra vendu par la société ZENECA COLOURS | 0,256 g |
| **- Gomme de guar modifiée par des groupements cationiques vendue sous la dénomination commerciale JAGUAR C 13 S par la société MAYHALL** | **1 g** |
| - Acide citrique qsp | pH 2,8 |
| - Eau déminéralisée qsp | 100 g |

On applique cette composition sur des cheveux humides, gris naturels à 90 % de blancs.
On laisse sécher les cheveux puis on les peigne.
Les cheveux sont colorés en marron léger et présentent un toucher agréable et naturel.

### EXEMPLE 3

On prépare la composition tinctoriale suivante :

| | |
|---|---|
| - N1,N4,N4-tri(β-hydroxyéthyl) 1,4-diamino 2-nitrobenzène (référencé HC BLUE 2 au Color Index 3ème édition) vendu par la société JAMES ROBINSON | 0,7 g |
| - 1-méthylamino 2-nitro 5-β,γ-dihydroxypropyloxybenzène | 0,3 g |
| **- Gomme de guar modifiée par des groupements cationiques vendue sous la dénomination commerciale JAGUAR C 13 S par la société MAYHALL** | **2 g** |
| - Ethanol | 15 g |
| - Eau déminéralisée qsp (pH spontané : 7,5) | 100 g |

On applique cette composition sur des mèches de cheveux humides, gris naturels à 90 % de blancs.
On laisse sécher les cheveux puis on les peigne.
Les cheveux sont colorés dans une nuance cendré léger et présentent un toucher agréable et naturel.

### EXEMPLE 4

On prépare la composition tinctoriale suivante :

| | |
|---|---|
| - N1,N4,N4-tri(β-hydroxyéthyl) 1,4-diamino 2-nitrobenzène (référencé HC BLUE 2 au Color Index 3ème édition) vendu par la société JAMES ROBINSON | 1,4 g |
| - 1-méthylamino 2-nitro 5-β,γ-dihydroxypropyloxybenzène | 0,6 g |
| **- Gomme de guar modifiée par des groupements cationiques vendue sous la dénomination commerciale JAGUAR C 13 S par la société MAYHALL** | **2 g** |
| - Ethanol | 15 g |
| - Eau déminéralisée qsp (pH spontané : 7,5) | 100 g |

On répartit environ 4 g de la composition tinctoriale ci-dessus sur une chevelure blonde ayant préalablement été lavée.
La chevelure est séchée au sèche-cheveux.
Les cheveux sont colorés en châtain clair cendré et présentent un toucher agréable et naturel.

### EXEMPLE 5 D'UTILISATION EN COLORATION D'OXYDATION

On prépare les compositions tinctoriales 1 et 2 suivantes :

### Composition 1 ne faisant pas partie de l'invention :

| | |
|---|---|
| - Paraphénylènediamine | 0,15 g |
| - Dichlorhydrate de 2,4-diamino 1-β-hydroxyéthyloxybenzène | 0,08 g |
| - 1-hydroxy 3-aminobenzène | 0,15 g |
| - Polymère d'acide acrylique réticulé vendu sous la dénomination commerciale CARBOPOL 934 par la société GOODRICH | 1 g |
| - Ethanol | 30 g |
| - Monoéthanolamine | 0,2 g |
| - Eau déminéralisée q.s.p. | 100 g |

### Composition 2 faisant partie de l'invention :

| | |
|---|---|
| - Paraphénylènediamine | 0,15 g |
| - Dichlorhydrate de 2,4-diamino 1-β-hydroxyéthyloxybenzène | 0,08 g |
| - 1-hydroxy 3-aminobenzène | 0,15 g |
| **- Gomme de guar modifiée par des groupements cationiques vendue sous la dénomination commerciale JAGUAR C 13 S par la société MAYHALL** | **1 g** |
| - Ethanol | 30 g |
| - Monoéthanolamine | 0,2 g |
| - Eau déminéralisée q.s.p. | 100 g |

Au moment de l'emploi, on mélange la composition tinctoriale 1 avec une quantité égale de la composition oxydante suivante :

| | |
|---|---|
| - Eau oxygénée qs | 10 volumes |
| - Acide orthophosphorique q.s. | pH 3 |
| - Polymère d'acide acrylique réticulé vendu sous la dénomination commerciale CARBOPOL 934 par la société GOODRICH | 1 g |
| - Eau déminéralisée q.s.p. | 100 g |

Au moment de l'emploi, on mélange la composition tinctoriale 2 avec une quantité égale de la composition oxydante suivante :

| | |
|---|---|
| - Eau oxygénée qs | 10 volumes |
| - Acide orthophosphorique q.s. | pH 3 |
| **- Gomme de guar modifiée par des groupements cationiques vendue sous la dénomination commerciale JAGUAR C 13 S par la société MAYHALL** | **1 g** |
| - Eau déminéralisée q.s.p. | 100 g |

Le pH des compositions résultantes appliquées sur les cheveux est de 6,5.
On applique ces mélanges sur des mèches de cheveux européens préalablement lavées au shampooing, à raison de 1 g de composition par gramme de cheveu. On laisse sécher les cheveux naturellement, puis on les délie avec un peigne.

Les cheveux teints avec la composition 1 utilisant un agent épaississant ne faisant pas partie de l'invention ont du mal à sécher, et une fois secs, présentent un toucher désagréable et inacceptable.
Seul un rinçage permet d'obtenir un toucher naturel. De plus, la mèche tache le papier sur lequel elle est frottée.

A l'inverse, les cheveux teints avec la composition 2 utilisant une gomme de guar modifiée par des groupements cationiques selon l'invention sèchent facilement et présentent une couleur intense. Leur toucher est agréable et naturel. Le passage de la mèche sur une feuille de papier ne laisse pas de trace.

### EXEMPLE 6

On prépare la composition tinctoriale suivante :

| | |
|---|---|
| - Paraphénylènediamine | 0,35 g |
| - Dichlorhydrate de 2,4-diamino 1-β-hydroxyéthyloxybenzène | 0,2 g |
| - 3-aminophénol | 0,35 g |
| **- Gomme de guar non-ionique modifiée par des groupements hydroxypropyle vendue sous la dénomination commerciale JAGUAR HP60 par la société MAYHALL** | **1,2 g** |
| - Ethanol | 25 g |
| - Monoéthanolamine | 1 g |
| - Eau déminéralisée q.s.p. | 100 g |

Au moment de l'emploi, on mélange la composition tinctoriale ci-dessus avec une quantité égale d'eau oxygénée à 20 volumes dont le pH est ajusté à 2,8 par de l'acide orthophosphorique.

Le mélange obtenu est appliqué sur une chevelure grise préalablement lavée et encore humide.
On laisse sécher les cheveux puis on les peigne.

Les cheveux sont teints en gris moyen et présentent un toucher agréable et naturel.

### EXEMPLE 7

On prépare la composition tinctoriale suivante :

| | |
|---|---|
| - Jaune Covanor W1603 (référencé pigment yellow 3 au Color Index 3ème édition) vendu par la société WACKHERR | 1 g |
| **- Gomme de guar modifiée par des groupements cationiques vendue sous la dénomination commerciale JAGUAR C 13 S par la société MAYHALL** | **1 g** |
| - Ethanol | 20 g |
| - Eau déminéralisée qsp (pH spontané : 6,2) | 100 g |

On applique cette composition sur des cheveux gris naturels à 90 % de blancs. On laisse sécher les cheveux puis on les peigne.

Les cheveux sont colorés en jaune et présentent un toucher agréable et naturel.

## Revendications

1. Utilisation d'une gomme de guar non-ionique et / ou d'une gomme de guar modifiée par des groupements cationiques, comme agent épaississant unique dans une composition de teinture non-rincée des fibres kératiniques humaines telles que les cheveux, renfermant au moins un colorant direct et/ou au moins un pigment, et/ou au moins un précurseur de colorant d'oxydation, dans le but d'obtenir des cheveux colorés brillants, doux au toucher, et ne tachant pas au frottement.

2. Utilisation selon la revendication 1 caractérisée par le fait que la gomme de guar non-ionique est modifiée par des groupements hydroxyalkyle en C₁-C₆.

3. Utilisation selon la revendication 2 caractérisée par le fait que les groupements hydroxyalkyle sont choisis parmi les groupements hydroxyméthyle, hydroxyéthyle, hydroxypropyle et hydroxybutyle.

4. Utilisation selon l'une quelconque des revendications 2 ou 3 caractérisée par le fait que la gomme de guar non-ionique présente un taux d'hydroxyalkylation variant entre 0,4 et 1,2.

5. Utilisation selon la revendication 1 caractérisée par le fait que les groupements cationiques de la gomme de guar sont des groupements trialkylammonium.

6. Utilisation selon la revendication 5 caractérisée par le fait que les groupements trialkylammonium sont choisis parmi les groupements triméthylammonium et triéthylammonium.

7. Utilisation selon l'une quelconque des revendications 5 ou 6 caractérisée par le fait que les groupements cationiques représentent moins de 30 % en poids par rapport au poids total de la gomme de guar modifiée.

8. Utilisation selon la revendication 7 caractérisée par le fait que les groupements cationiques représentent de 5 à 20 % en poids par rapport au poids total de la gomme de guar modifiée.

9. Utilisation selon l'une quelconque des revendications 5 à 8 caractérisée par le fait que la gomme de guar est modifiée par du chlorure de 2,3-époxypropyl triméthylammonium.

10. Utilisation selon l'une quelconque des revendications 1 à 9 caractérisée par le fait que la quantité de gomme de guar non-ionique et / ou de gomme de guar modifiée par des groupements cationiques est comprise entre 0,1 et 3 % en poids par rapport au poids total de la composition tinctoriale .

11. Utilisation selon la revendication 10 caractérisée par le fait que la quantité de gomme de guar non-ionique et / ou de gomme de guar modifiée par des groupements cationiques est comprise entre 0,25 et 2,5 % en poids par rapport au poids total de la composition tinctoriale .

12. Utilisation selon l'une quelconque des revendications 1 à 11 caractérisée par le fait que les colorants directs sont choisis parmi les dérivés nitrés de la série benzénique, les colorants azoïques, les colorants anthraquinoniques, les indamines, indoanilines et indophénols, les colorants acides, les colorants naturels.

13. Utilisation selon l'une quelconque des revendications 1 à 12 caractérisée par le fait que les pigments sont choisis parmi les pigments minéraux, organiques ou nacrés.

14. Utilisation selon l'une quelconque des revendications 1 à 13 caractérisée par le fait que les précurseurs de colorants d'oxydation sont choisis parmi les diamines ou aminophénols comportant des groupements fonctionnels amino et hydroxy en position para ou ortho.

15. Utilisation selon la revendication 14 caractérisée par le fait que la composition contenant au moins un précurseur de colorant d'oxydation contient en outre au moins un coupleur choisi parmi les métadiamines aromatiques, les métaaminophénols et les métadiphénols.

16. Utilisation selon l'une quelconque des revendications 1 à 15 caractérisée par le fait que les compositions contiennent des précurseurs indoliques.

17. Utilisation selon la revendication 16 caractérisée par le fait que les précurseurs indoliques sont choisis parmi le 5,6-dihydroxyindole et ses dérivés et les 6- et 7-monohydroxyindoles.

18. Utilisation selon l'une quelconque des revendications 1 à 17 caractérisée par le fait que la composition destinée à la teinture non rincée des fibres kératiniques contient en outre un milieu aqueux constitué par de l'eau ou un mélange d'eau et d'un solvant organique.

19. Utilisation selon l'une quelconque des revendications 1 à 18 caractérisée par le fait que la composition renferme au moins un adjuvant choisi parmi les agents conservateurs, les agents séquestrants, les agents anti-oxydants, les parfums, les tensioactifs non-ioniques, les filtres solaires.

## Claims

1. Use of a nonionic guar gum and/or of a guar gum modified with cationic groups, as sole thickening agent in a non-rinsed composition for dyeing human keratin fibres such as the hair, containing at least one direct dye and/or at least one pigment and/or at least one oxidation dye precursor for the purpose of obtaining shiny dyed hair which feels soft and does not leave stains when rubbed.

2. Use according to Claim 1, characterized in that the nonionic guar gum is modified with C₁-C₆ hydroxyalkyl groups.

3. Use according to Claim 2, characterized in that the hydroxyalkyl groups are chosen from hydroxymethyl, hydroxyethyl, hydroxypropyl and hydroxybutyl groups.

4. Use according to either of Claims 2 and 3, characterized in that the nonionic guar gum has a degree of hydroxyalkylation ranging between 0.4 and 1.2.

5. Use according to Claim 1, characterized in that the cationic groups of the guar gum are trialkylammonium groups.

6. Use according to Claim 5, characterized in that the trialkylammonium groups are chosen from trimethylammonium and triethylammonium groups.

7. Use according to either of Claims 5 and 6, characterized in that the cationic groups represent less than 30% by weight relative to the total weight of the modified guar gum.

8. Use according to Claim 7, characterized in that the cationic groups represent from 5 to 20% by weight relative to the total weight of the modified guar gum.

9. Use according to any one of Claims 5 to 8, characterized in that the guar gum is modified with 2,3-epoxypropyltrimethylammonium chloride.

10. Use according to any one of Claims 1 to 9, characterized in that the amount of nonionic guar gum and/or of guar gum modified with cationic groups is between 0.1 and 3% by weight relative to the total weight of the dye composition.

11. Use according to Claim 10, characterized in that the amount of nonionic guar gum and/or of guar gum modified with cationic groups is between 0.25 and 2.5% by weight relative to the total weight of the dye composition.

12. Use according to any one of Claims 1 to 11, characterized in that the direct dyes are chosen from nitro derivatives of the benzene series, azo dyes, anthraquinone dyes, indamines, indoanilines and indophenols, acidic dyes and natural dyes.

13. Use according to any one of Claims 1 to 12, characterized in that the pigments are chosen from inorganic, organic or pearlescent pigments.

14. Use according to any one of Claims 1 to 13, characterized in that the oxidation dye precursors are chosen from diamines or aminophenols containing amino and hydroxyl functional groups in the para or ortho position.

15. Use according to Claim 14, characterized in that the composition containing at least one oxidation dye precursor additionally contains at least one coupler chosen from aromatic meta-diamines, meta-aminophenols and meta-diphenols.

16. Use according to any one of Claims 1 to 15, characterized in that the compositions contain indole precursors.

17. Use according to Claim 16, characterized in that the indole precursors are chosen from 5,6-dihydroxyindole and derivatives thereof and 6- and 7-monohydroxyindoles.

18. Use according to any one of Claims 1 to 17, characterized in that the composition intended for the non-rinsed dyeing of keratin fibres additionally contains an aqueous medium consisting of water or a mixture of water and an organic solvent.

19. Use according to any one of Claims 1 to 18, characterized in that the composition contains at least one adjuvant chosen from preserving agents, sequestering agents, antioxidants, fragrances, nonionic surfactants and sunscreens.

## Patentansprüche

1. Verwendung eines nichtionischen Guargummis und/oder eines mit kationischen Gruppen modifizierten Guargummis als einziges Verdickungsmittel in einer Zusammensetzung zum Färben von menschlichen Keratinfasern, wie den Haaren, die nicht ausgespült wird und die mindestens einen Direktfarbstoff und/oder mindestens ein Pigment und/oder mindestens ein Farbstoffvorprodukt eines Oxidationsfarbstoffes enthält, um zu erreichen, daß die gefärbten Haare glänzen, sich geschmeidig anfühlen und beim Abreiben keine Flecken hinterlassen.

2. Verwendung nach Anspruch 1, dadurch gekennzeichnet, daß das nichtionische Guargummi mit C₁₋₆-Hydroxyalkylgruppen modifiziert ist.

3. Verwendung nach Anspruch 2, dadurch gekennzeichnet, daß die Hydroxyalkylgruppen unter Hydroxymethylgruppen, Hydroxyethylgruppen, Hydroxypropylgruppen und Hydroxybutylgruppen ausgewählt sind.

4. Verwendung nach Anspruch 2 oder 3, dadurch gekennzeichnet, daß das nichtionische Guargummi einen Oxyalkylierungsgrad aufweist, der im Bereich von 0,4 bis 1,2 liegt.

5. Verwendung nach Anspruch 1, dadurch gekennzeichnet, daß die kationischen Gruppen des Guargummis Trialkylammoniumgruppen sind.

6. Verwendung nach Anspruch 5, dadurch gekennzeichnet, daß die Trialkylammoniumgruppen unter Trimethylammoniumgruppen und Triethylammoniumgruppen ausgewählt sind.

7. Verwendung nach Anspruch 5 oder 6, dadurch gekennzeichnet, daß die kationischen Gruppen weniger als 30 Gew.-%, bezogen auf das Gesamtgewicht des modifizierten Guargummis, ausmachen.

8. Verwendung nach Anspruch 7, dadurch gekennzeichnet, daß die kationischen Gruppen 5 bis 20 Gew.-%, bezogen auf das Gesamtgewicht des modifizierten Guargummis, ausmachen.

9. Verwendung nach einem der Ansprüche 5 bis 8, dadurch gekennzeichnet, daß das Guargummi mit 2,3-Epoxypropyltrimethylammoniumchlorid modifiziert ist.

10. Verwendung nach einem der Ansprüche 1 bis 9, dadurch gekennzeichnet, daß der Mengenanteil des nichtionischen Guargummis und/oder des mit kationischen Gruppen modifizierten Guargummis im Bereich von 0,1 bis 3 Gew.-%, bezogen auf das Gesamtgewicht der Zusammensetzung zum Färben, liegt.

11. Verwendung nach Anspruch 10, dadurch gekennzeichnet, daß der Mengenanteil des nichtionischen Guargummis und/oder des mit kationischen Gruppen modifizierten Guargummis im Bereich von 0,25 bis 2,5 Gew.-%, bezogen auf das Gesamtgewicht der Zusammensetzung zum Färben, liegt.

12. Verwendung nach einem der Ansprüche 1 bis 11, dadurch gekennzeichnet, daß die Direktfarbstoffe unter Nitroderivaten aus der Benzolgruppe, Azofarbstoffen, Anthrachinonfarbstoffen, Indaminen, Indoanilinen und Indophenolen, sauren Farbstoffen oder natürlichen Farbstoffen ausgewählt sind.

13. Verwendung nach einem der Ansprüche 1 bis 12, dadurch gekennzeichnet, daß die Pigmente unter anorganischen Pigmenten, organischen Pigmenten oder Perlglanzpigmenten ausgewählt sind.

14. Verwendung nach einem der Ansprüche 1 bis 13, dadurch gekennzeichnet, daß die Farbstoffvorprodukte von Oxidationsfarbstoffen unter Diaminen oder Aminophenolen ausgewählt sind, die funktionelle Aminogruppen und Hydroxygruppen in Parastellung oder in Orthostellung tragen.

15. Verwendung nach Anspruch 14, dadurch gekennzeichnet, daß die Zusammensetzung, die mindestens ein Farbstoffvorprodukt eines Oxidationsfarbstoffes enthält, ferner mindestens einen Kuppler umfaßt, der unter aromatischen m-Diaminen, m-Aminophenolen und m-Dihydroxybenzolen ausgewählt ist.

16. Verwendung nach einem der Ansprüche 1 bis 15, dadurch gekennzeichnet, daß die Zusammensetzungen Indolprecursor enthalten.

17. Verwendung nach Anspruch 16, dadurch gekennzeichnet, daß die Indolprecursor unter 5,6-Dihydroxyindol und seinen Derivaten, 6-Monohydroxyindol und 7-Monohydroxyindol ausgewählt sind.

18. Verwendung nach einem der Ansprüche 1 bis 17, dadurch gekennzeichnet, daß die Zusammensetzung zum Färben von Keratinfasern, die nicht ausgespült wird, ferner ein wäßriges Medium enthält, das aus Wasser oder aus einem Gemisch von Wasser und einem organischen Lösungsmittel besteht.

19. Verwendung nach einem der Ansprüche 1 bis 18, dadurch gekennzeichnet, daß die Zusammensetzung mindestens einen Hilfsstoff enthält, der unter Konservierungsmitteln, Maskierungsmitteln, Antioxidationsmitteln, Parfüms, nichtionischen grenzflächenaktiven Mitteln und Sonnenschutzfiltern ausgewählt ist.
